(19) Europäisches Patentamt
European Patent Office
Office européen des brevets

(11) Numéro de publication: **0 027 085**
**B1**

(12) # FASCICULE DE BREVET EUROPEEN

(45) Date de publication du fascicule du brevet: **14.12.83**

(21) Numéro de dépôt: **80401411.6**

(22) Date de dépôt: **03.10.80**

(51) Int. Cl.³: **C 07 D 513/04,**
A 61 K 31/54 //(C07D513/04, 277/00, 265/00)

(54) **Nouveaux dérivés de la thiazolo (3,2-c) benzoxazine-1,3, leur préparation et les médicaments qui les contiennent.**

(30) Priorité: **05.10.79 FR 7924833**

(43) Date de publication de la demande:
**15.04.81 Bulletin 81/15**

(45) Mention de la délivrance du brevet:
**14.12.83 Bulletin 83/50**

(84) Etats contractants désignés:
**AT BE CH DE FR GB IT LI LU NL SE**

(56) Documents cités:
**CHEMICAL ABSTRACTS, vol. 88, no. 7 13-02-1978, page 551, abrégé no. 50879f Columbus, Ohio, US**

(73) Titulaire: **RHONE-POULENC SANTE**
**Les Miroirs 18 Avenue d'Alsace**
**F-92400 Courbevoie Cedex (FR)**

(72) Inventeur: **Berger, Christian**
**26 A, rue Paul Rivet**
**F-92350 Le Plessis Robinson (FR)**
Inventeur: **Farge, Daniel**
**30, rue des Pins Sylvestres**
**F-94320 Thiais (FR)**
Inventeur: **Moutonnier, Claude**
**3, rue Auguste Rodin**
**F-92350 Le Plessis Robinson (FR)**
Inventeur: **Wolff, Gérard**
**7, rue Jeanne d'Arc**
**F-94320 Thiais (FR)**

(74) Mandataire: **Gaumont, Robert et al,**
**RHONE-POULENC RECHERCHES Service Brevets Pharma 25, Quai Paul Doumer**
**F-92408 Courbevoie Cedex (FR)**

**Le dossier contient des informations techniques présentées postérieurement au dépôt de la demande et ne figurant pas dans le présent fascicule.**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**EP 0 027 085 B1**

Nouveaux dérivés de la thiazolo (3,2-c) benzoxazine-1,3, leur préparation et les médicaments qui les contiennent

La présente invention concerne de nouveaux dérivés de la thiazolo[3,2-c]benzoxazine-1,3 de formule générale:

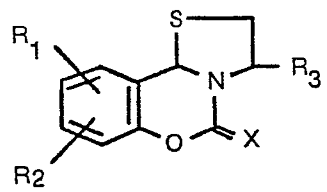

(I)

éventuellement leurs sels, leur préparation et les médicaments qui les contiennent.

Dans la formule générale (I), le symbole X représente un atome d'oxygène ou de soufre, les symboles $R_1$ et $R_2$ (qui sont identiques ou différents) représentent chacun un atome d'hydrogène ou d'halogène ou un radical alcoyle, alcoyloxy, alcoylthio ou trifluorométhyle, en position -7, -8, -9 ou -10, et le symbole $R_3$ représente un atome d'hydrogène ou un radical carboxy.

Il est entendu que les portions ou radicaux alcoyles sont droits ou ramifiés et contiennent 1 à 4 atomes de carbone et que les produits pour lesquels $R_3$ est autre qu'un atome d'hydrogène dérivent des formes L, D et D, L de la cystéine.

La demande de brevet japonais publiée sous le N° 77 95696 décrit des dérivés de thiazolobenzoxazines répondant à la formule générale:

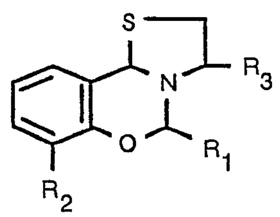

dans laquelle $R_1$ représente un radical alcoyle, phényle ou furyle. Ces produits sont doués d'activité analgésique et anti-inflammatoire.

Les thiazolobenzoxazines ci-dessus sont obtenues par un procédé qui ne permettrait pas de préparer des oxo (ou thioxo)-5 thiazolobenzoxazines.

Selon l'invention, les produits de formule générale (I) peuvent être obtenus par action d'un produit de formule générale:

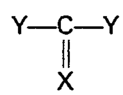

(II)

(dans laquelle X est défini comme précédemment et les symboles Y, qui sont identiques, représentent des atomes de chlore ou des radicaux imidazolyle, benzimidazolyle, triazolyle, benzotriazolyle ou pyrazolyle), sur un dérivé de la thiazolidine [éventuellement en équilibre avec sa forme "imine", selon J. J. PESEK et J. H. FROST, Tetrahedron, *31* 907 (1975)] répondant à la formule générale:

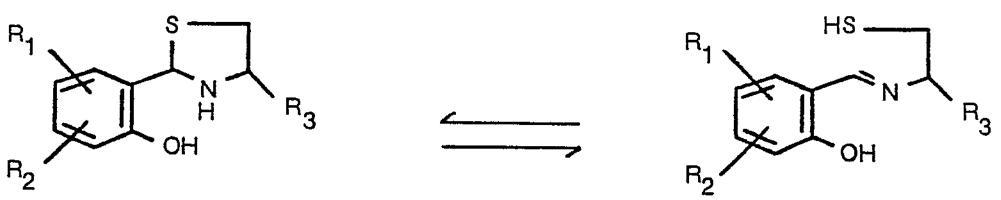

(III)

dans laquelle $R_1$, $R_2$ et $R_3$ sont définis comme précédemment.

On opère généralement dans un solvant organique aromatique (par exemple benzène, toluène), dans un solvant chloré (par exemple chlorure de méthylène, chloroforme, dichloro-1,2 éthane) ou dans un éther (par exemple éther éthylique, tétrahydrofuranne ou dioxanne) à une température comprise entre −20°C et la température de reflux du mélange réactionnel, et en présence d'une base telle qu'une base organique azotée (par exemple triéthylamine) lorsque l'on utilise un réactif de formule générale (II) dans laquelle Y représente un atome de chlore.

Les produits de formule générale (II), dans laquelle Y est autre qu'un atome de chlore, peuvent être préparés selon la méthode décrite par C. LARSEN, K. STELIOU et D. N. HARPP, J. Org. Chem. *43,* 2, 337 (1978).

Les dérivés de la thiazolidine de formule générale (III) peuvent être obtenus par application de la méthode décrite par M. FATOME et coll., Chim. Ther., *5,* 312 (1970), ou de la méthode décrite dans The Chemistry of penicillins, p. 962, Princeton University Press (1949), Princeton, New Jersey (U.S.A.) à partir de l'aldéhyde salicylique ou d'un de ses dérivés et de cystéamine ou de L, D ou D,L cystéine.

Il n'est pas absolument nécessaire d'isoler le dérivé de la thiazolidine de formule générale (III) pour mettre en oeuvre l'action du produit de formule générale (II).

Les dérivés substitués de l'aldéhyde salicylique peuvent être préparés par application de la méthode décrite par G. CASIRAGHI et coll., J. C. S. PERKIN I, 318 (1978).

Si on le désire, les composés de la présente invention peuvent être purifiés par des méthodes physiques telles que cristallisation ou chromatographie.

Les nouveaux produits selon l'invention peuvent éventuellement être transformés en sels métalliques ou en sels d'addition avec une base azotée lorsque $R_3$ représente un radical carboxy. Ces sels peuvent être obtenus par action d'une base métallique (notamment alcaline ou alcalino-terreuse), de l'ammoniac ou d'une base organique azotée, dans un solvant approprié tel qu'un alcool, un éther ou l'eau, ou par réaction d'échange avec un sel d'un acide organique. Le sel formé précipite après concentration éventuelle de sa solution; il est séparé par filtration ou décantation.

Les nouveaux produits selon l'invention et leurs sels présentent des propriétés pharmacologiques remarquables. Ce sont des agents analgésiques particuliérement intéressants. Certains d'entre eux se sont également montrés anti-inflammatoires et anti-thermiques.

L'activité analgésique se manifeste chez le rat à des doses comprises entre 5 et 200 mg/kg par voie orale dans la technique de E. SIEGMUND et coll., Proc. Soc. Exp. Biol. Med., *95,* 729 (1957).

L'activité anti-inflammatoire se manifeste chez le rat à des doses comprises entre 25 et 250 mg/kg par voie orale selon la technique de K. F. BENITZ et L. M. HALL [Arch. Int. Pharmacodyn., *144,* 185 (1963)].

L'activité anti-thermique se manifeste chez le rat à des doses comprises entre 5 et 50 mg/kg par voie orale dans la technique de J. J. LOUX et coll., Toxicol. Appl. Pharmacol., *22,* 674 (1972).

Par ailleurs la toxicité aiguë des produits selon l'invention, exprimée par leur $DL_{50}$, est supérieure à 900 mg/kg par voie orale chez la souris.

De particulière valeur sont les produits dans la formule desquels X représente un atome d'oxygène ou de soufre, $R_1$ représente un atome d'hydrogène ou un radical alcoyle ou alcoyloxy (tels que définis ci-avant) en position -7 ou -9, $R_2$ représente un atome d'hydrogène et $R_3$ représente un atome d'hydrogène ou un radical carboxy.

Parmi ces composés on préfère ceux pour lesquels $R_1$ et $R_2$ représentent des atomes d'hydrogène.

Pour l'emploi médicinal il est fait usage des nouveaux composés soit à l'état d'acide, soit à l'état de sels pharmaceutiquement acceptables, c'est-à-dire non toxiques aux doses d'utilisation, lorsque $R_3$ représente un radical carboxy.

Les exemples suivants, donnés à titre non limitatif, montrent comment l'invention peut être mise en pratique.

Exemple 1

On dissout 85,4 g d'(hydroxy-2 phényl)-2 thiazolidine dans 300 cm3 de tétrahydrofuranne. On ajoute en 10 minutes une solution de 93,3 g de N,N'-thiocarbonyldiimidazole dans 700 cm3 de tétrahydrofuranne. Le mélange est chauffé jusqu'à reflux pendant 20 minutes. La solution est alors refroidie à 20°C et concentrée à sec sous pression réduite (20 mm de mercure; 2,67 kPa) à 30°C. Le résidu est dissous dans 1200 cm3 de chlorure de méthylène. La solution obtenue est lavée avec deux fois 1500 cm3 d'eau distillée et séchée sur sulfate de sodium. La solution est filtrée en présence de noir décolorant et concentrée à sec sous pression réduite (100 mm de mercure; 13,3 kPa) à 20°C. Le résidu est recristallisé dans 1000 cm3 d'éthanol absolu. Les cristaux sont séparés par filtration et séchés sous pression réduite (0,5 mm de mercure; 0,07 kPa) à 40°C. On obtient ainsi 51,6 g de thioxo-5 tétrahydro-2,3,5,10b thiazolo [3,2-c] benzoxazine-1,3 sous forme de cristaux blancs, F. inst. (Kofler) = 127°C.

Exemple 2

On dissout 36,2 g d'(hydroxy-2 phényl)-2 thiazolidine dans 400 cm3 de chlorure de méthylène. On ajoute 35,6 g de N,N'-thiocarbonyldiimidazole et laisse réagir sous agitation pendant 14 heures. La solution est filtrée et le filtrat est concentré à sec sous pression réduite (100 mm de mercure; 13,3 kPa) à 20°C. Le résidu est dissous dans 350 cm3 d'acétate d'éthyle. La solution obtenue est lavée avec deux fois 300 cm3 d'eau, puis séchée sur sulfate de sodium et filtrée en présence de noir décolorant. Le filtrat est concentré à sec sous pression réduite (20 mm de mercure; 2,67 kPa) à 40°C. Le résidu est repris avec 60 cm3 d'éthanol. Les cristaux sont essorés et séchés sous pression réduite (0,5 mm de mercure; 0,07 kPa) à 40°C. On obtient ainsi 9,9 g de thioxo-5 tétrahydro-2,3,5,10b thiazolo [3,2-c] benzoxazine-1,3 dont les caractéristiques sont identiques à celles du produit de l'exemple 1.

Exemple 3

A une solution de 4,2 cm3 de thiophosgène dans 50 cm3 de tétrahydrofuranne on ajoute, goutte à goutte de façon à maintenir une température de 30°C, une solution de 10 g d'(hydroxy-2 phényl)-2 thiazolidine et de 15,4 cm3 de triéthylamine dans 50 cm3 de tétrahydrofuranne. Le mélange est encore agité pendant 30 minutes à 20°C après la fin de l'addition. Le solvant est évaporé à sec sous pression réduite (20 mm de mercure; 2,67 kPa) à 40°C et le résidu est redissous dans un mélange de 100 cm3 d'acétate d'éthyle et 100 cm3 d'eau. La phase aqueuse est séparée par décantation et la phase organique est lavée avec 100 cm3 de solution aqueuse saturée de bicarbonate de sodium puis avec 100 cm3 d'eau distillée. La phase organique est séchée sur sulfate de sodium et filtrée en présence de noir décolorant. Le solvant est évaporé à sec sous pression réduite (20 mm de mercure; 2,67 kPa) à 40°C. On obtient 7,3 g de thioxo-5 tétrahydro-2,3,5,10b thiazolo [3,2-c] benzoxazine-1,3 dont les caractéristiques sont identiques à celles du produit de l'exemple 1.

Exemple 4

On ajoute 14 cm3 de triéthylamine à une suspension de 11,6 g de chlorhydrate de cystéamine dans 80 cm3 de dichloro-1,2 éthane. Le mélange est chauffé à reflux puis on ajoute, en 2 minutes, 10,5 cm3 d'aldéhyde salicylique. Le mélange est chauffé à reflux pendant 15 minutes et l'eau formée est entraînée par distillation azéotropique. On ajoute alors une solution de 22,2 g de N,N'-thiocarbonyldiimidazole dans 80 cm3 de dichloro-1,2 éthane en 2 minutes. Le mélange est ensuite maintenu à reflux pendant 10 minutes puis refroidi à 20°C. La solution obtenue est lavée avec trois fois 150 cm3 d'eau distillée. La phase organique est séchée sur sulfate de sodium, filtrée en présence de noir décolorant puis concentrée sous pression réduite (20 mm de mercure; 2,67 kPa) à 40°C jusqu'à un volume de 20 cm3. Cette solution est filtrée sur une colonne (diamètre 3 cm) contenant 60 g de silice. On élue avec 200 cm3 de dichloro-1,2 éthane. Le filtrat et l'éluat sont concentrés à sec sous pression réduite (20 mm de mercure; 2,67 kPa) à 40°C et le résidu est dissous dans 15 cm3 d'acétate d'éthyle à reflux. La solution est refroidie et maintenue à 4°C pendant 1 heure. Les cristaux sont séparés par filtration. On obtient ainsi 2,2 g de thioxo-5 tétrahydro-2,3,5,10b thiazolo [3,2-c] benzoxazine-1,3 dont les caractéristiques sont identiques à celles du produit de l'exemple 1.

Exemple 5

On ajoute 22,7 g de N,N'-thiocarbonyldiimidazole à une solution de 25 g de (chloro-5 hydroxy-2 phényl)-2 thiazolidine dans 270 cm3 de tétrahydrofuranne. La solution obtenue est chauffée à reflux pendant 25 minutes. La solution est ensuite refroidie et maintenue à 0°C pendant 1 heure. Les cristaux obtenus sont séparés par filtration, lavés avec deux fois 30 cm3 de tétrahydrofuranne froid et séchés sous pression réduite (0,5 mm de mercure; 0,07 kPa) à 40°C. On obtient ainsi 11 g de chloro-9 thioxo-5 tétrahydro-2,3,5,10b thiazolo [3,2-c] benzoxazine-1,3 sous forme de cristaux blancs.

F. inst. (Kofler) = 261°C.

Exemple 6

On ajoute 23,1 g de N,N'-thiocarbonyldiimidazole à une solution de 25 g d'(hydroxy-2 méthoxy-3 phényl)-2 thiazolidine dans 270 cm3 de tétrahydrofuranne. La solution obtenue est chauffée à reflux pendant 25 minutes. La solution est refroidie et maintenue à 0°C pendant 2 heures. Les cristaux formés sont séparés par filtration puis lavés avec deux fois 50 cm3 de tétrahydrofuranne froid et séchés sous pression réduite (0,5 mm de mercure; 0,07 kPa) à 40°C. On obtient ainsi 12,5 g de cristaux blancs de méthoxy-7 thioxo-5 tétrahydro-2,3,5,10b thiazolo [3,2-c] benzoxazine-1,3.

F. inst. (Kofler) = 173°C.

Exemple 7

On ajoute 25,3 g de N,N'-thiocarbonyldiimidazole à une solution de 30 g d'(hydroxy-2 méthoxy-5 phényl)-2 thiazolidine dans 300 cm3 de tétrahydrofuranne. Le mélange est agité, chauffé à reflux pendant 10 minutes, puis refroidi à 20°C. Le solvant est évaporé sous pression réduite (20 mm de mercure; 2,67 kPa) à 40°C et le résidu est dissous dans 300 cm3 d'acétate d'éthyle. On lave avec 3 fois 200 cm3 d'eau distillée puis sèche la phase organique sur sulfate de sodium et filtre la solution. Le solvant est évaporé sous pression réduite (20 mm de mercure; 2,67 kPa) à 40°C et le résidu obtenu est chromatographié sur une colonne (diamètre 6 cm) contenant 500 g de silice. On élue avec 4000 cm3 de mélange acétate d'éthyle — cyclohexane (3—7 en volumes) en recueillant des fractions de 500 cm3. Les fractions 4 à 7 sont réunies et le solvant évaporé à sec sous pression réduite (20 mm de mercure; 2,67 kPa) à 40°C. Le produit obtenu est repris avec 100 cm3 d'ether et les cristaux blancs insolubles sont séparés par filtration. On obtient ainsi 7,5 g de méthoxy-9 thioxo-5 tétrahydro-2,3,5,10b thiazolo [3,2-c] benzoxazine-1,3.

F. inst. (Kofler) = 134°C.

Exemple 8

On dissout 45,25 g d'(hydroxy-2 phényl)-2 thiazolidine dans 500 cm3 de chlorure de méthylène. On ajoute 40,5 g de N,N'-carbonyldiimidazole et laisse réagir sous agitation pendant 14 heures. La

solution est concentrée à sec sous pression réduite (100 mm de mercure; 13,3 kPa) à 20°C. Le résidu est repris avec 500 cm3 d'acétate d'éthyle et la solution obtenue est lavée avec deux fois 500 cm3 d'eau distillée. La phase organique est séchée sur sulfate de sodium et filtrée en présence de noir décolorant puis concentrée à sec sous pression réduite (20 mm de mercure; 2,67 kPa) à 40°C. Le résidu huileux obtenu est chromatographié sur une colonne (diamètre 60 mm) contenant 500 g de silice. On élue successivement avec des mélanges d'acétate d'éthyle et de cyclohexane de concentration croissante en acétate d'éthyle (10—90 en volumes: 4000 cm3 puis 20—80 en volumes: 4000 cm3). On recueille des fractions de 350 cm3. Les fractions 11 à 13 sont réunies et concentrées à sec sous pression réduite (0,5 mm de mercure; 0,07 kPa) à 40°C. On obtient ainsi 12 g de cristaux blancs d'oxo-5 tétrahydro-2,3,5,10b thiazolo [3,2,-c] benzoxazine-1,3.

F. inst. (Kofler) = 77°C.

Exemple 9

On ajoute 28 cm3 de triéthylamine à une suspension de 45 g de L-carboxy-4 (hydroxy-2-phényl)-2 thiazolidine dans 600 cm3 de chlorure de méthylène puis agite le mélange pendant 20 minutes à 20°C. On ajoute 71,2 g de N,N'-thiocarbonyldiimidazole et agite le mélange à 20°C pendant 18 heures. Le solvant est alors évaporé sous pression réduite (100 mm de mercure; 13,3 kPa) à 20°C et le résidu est repris avec 500 cm3 d'eau distillée. On recouvre la phase aqueuse avec 500 cm3 d'acétate d'éthyle et agite le mélange. Le pH du mélange est ramené à 2 par addition d'acide chlorhydrique 4 N. La phase organique est décantée et la phase aqueuse est extraite avec 3 fois 100 cm3 d'acétate d'éthyle. Les phases organiques sont réunies et extraites avec 500 cm3 de solution aqueuse saturée de bicarbonate de sodium. La phase aqueuse est recouverte avec 100 cm3 d'acétate d'éthyle et acidifiée à pH = 2 par addition d'acide chlorhydrique 4 N. La phase organique est séparée par décantation, séchée sur sulfate de sodium puis filtrée en présence de noir décolorant et enfin concentrée à sec sous pression réduite (20 mm de mercure; 2,67 kPa) à 40°C. Le résidu huileux obtenue est dissous dans 600 cm3 le mélange éthanol-eau (1—1 en volumes). La solution est placée à 4°C pendant 1 heure puis les cristaux sont séparés par filtration et séchés. On obtient ainsi 9,2 g de carboxy-3 thioxo-5 tétrahydro-2,3,5,10b thiazolo [3,2-c] benzoxazine-1,3 sous forme de cristaux blancs.

F. inst. (Kofler) = 225°C (déc.)

On dissout 203 mg de carboxy-3 thioxo-5 tétrahydro-2,3,5,10b thiazolo [3,2-c] benzoxazine-1,3 dans une solution de 64 mg de bicarbonate de sodium dans 7,6 cm3 d'eau. La solution obtenue est évaporée à sec sous pression réduite (20 mm de mercure; 2,67 kPa) à 40°C et le résidu est repris avec 25 cm3 d'éther diéthylique. On filtre la suspension et sèche le solide sous pression réduite (0,5 mm de mercure; 0,07 kPa) à 20°C. On obtient ainsi 190 mg du sel de sodium de carboxy-3 thioxo-5 tétrahydro-2,3,5,10b thiazolo [3,2-c] benzoxazine-1,3 sous la forme d'une poudre blanche.

Spectre infra-rouge (KBr): bandes caractéristiques ($cm^{-1}$): 1615, 1460 et 750.

Exemple 10

On ajoute 24 cm3 de triéthylamine à une suspension de 38,4 g de L-carboxy-4 (hydroxy-2 phényl)-2 thiazolidine dans 200 cm3 de tétrahydrofuranne. Le mélange est chauffé à reflux jusqu'à dissolution complète puis on ajoute, en 5 minutes, une soluton de 60,7 g de N,N'-thiocarbonyldiimidazole dans 300 cm3 de tétrahydrofuranne. Le mélange est maintenu à reflux pendant 10 minutes puis refroidi et concentré à sec sous pression réduite (20 mm de mercure; 2,67 kPa) à 40°C. Le résidu obtenu est repris avec 200 cm3 de solution aqueuse saturée de bicarbonate de sodium. On lave avec 2 fois 200 cm3 d'acétate d'éthyle puis recouvre la phase aqueuse avec 200 cm3 d'acétate d'éthyle et acidifie à pH = 2 par addition d'acide chlorhydrique 4 N. La phase organique est séparée par décantation. La phase aqueuse est encore extraite avec 100 cm3 d'acétate d'éthyle. On réunit les phases organiques, sèche sur sulfate de sodium, filtre en présence de noir décolorant et concentre à sec sous pression réduite (20 mm de mercure; 2,67 kPa) à 40°C. On obtient ainsi 7,5 g de carboxy-3 thioxo-5 tétrahydro-2,3,5,10b thiazolo [3,2-c] benzoxazine-1,3.

Exemple 11

On dissout 35 g d'(hydroxy-2 méthyl-5 phényl)-2 thiazolidine dans 350 cm3 de chlorure de méthylène. On ajoute 35,1 g de N,N'-thiocarbonyldiimidazole et chauffe le mélange à reflux pendant 1 heure. La solution obtenue est lavée avec 500 cm3 d'eau distillée puis séchée sur sulfate de magnésium et filtrée en présence de noir décolorant. Le filtrat est concentré à sec sous pression réduite (20 mm de mercure; 2,67 kPa) à 40°C. Le résidu est repris avec 100 cm3 d'acétate d'éthyle. Les cristaux sont essorés et séchés sous pression réduite (0,5 mm de mercure; 0,07 kPa) à 40°C. On obtient ainsi 8,9 g de méthyl-9 thioxo-5 tétrahydro-2,3,5,10b thiazolo [3,2-c] benzoxazine-1,3 sous la forme de cristaux blancs, F. inst. (Kofler): 159°C.

Exemple 12

En procédant de la façon décrite dans l'exemple 11, avec 15 g de (fluoro-5 hydroxy-2 phényl)-2 thiazolidine et 14,7 g de N,N'-thiocarbonyldiimidazole dans 150 cm3 de chlorure de méthylène on obtient 7,5 g de fluoro-9 thioxo-5 tétrahydro-2,3,5,10b thiazolo [3,2-c] benzoxazine-1,3 sous la forme de cristaux blancs, F. inst. (Kofler): 180°C.

Les médicaments constitués par un dérivé de formule générale (I) à l'état pur, éventuellement sous forme d'acide libre ou de sel pharmaceutiquement acceptable, et les compostiions pharmaceutiques qui le contiennent en association avec au moins un diluant ou adjuvant compatible et pharmaceutiquement acceptable, constituent un autre objet de la présente invention. Ces compositions peuvent être employées par voie orale, rectale, parentérale ou topique.

Comme compositions solides pour administration orale peuvent être utilisés des comprimés, pilules, poudres, gélules ou granulés. Dans ces compositions, le produit actif selon l'invention est mélangé à un ou plusieurs diluants inertes tels que saccharose, lactose ou amidon. Ces compositions peuvent également comprendre des substances autres que les diluants, par exemple un lubrifiant tel que le stéarate de magnésium.

Comme compositions liquides pour administration orale peuvent être utilisés des émulsions pharmaceutiquement acceptables, des solutions, des suspensions, des sirops et des élixirs contenant des diluants inertes tels que l'huile de paraffine. Ces compositions peuvent également comprendre des substances autres que les diluants, par exemple des produits mouillants, édulcorants ou aromatisants.

Les compositions selon l'invention pour administration parentérale peuvent être des solutions stériles aqueuses ou non aqueuses, des suspensions ou des émulsions. Comme solvant ou véhicule, on peut employer l'eau, le propylèneglycol, un polyéthylèneglycol, des huiles végétales en particulier l'huile d'olive, et des esters organiques injectables, par exemple l'oléate d'éthyle. Ces compositions peuvent également contenir des adjuvants, en particulier des agents mouillants, émulsifiants ou dispersants. La stérilisation peut se faire de plusieurs façons, par exemple à l'aide d'un filtre bactériologique, en incorporant à la composition des agents stérilisants, par irradiation ou par chauffage. Elles peuvent être préparées sous forme de compositions solides stériles qui peuvent être dissoutes au moment de l'emploi dans un milieu stérile injectable.

Les compositions pour administration rectale sont des suppositoires qui peuvent contenir, outre le produit actif, des excipients tels que le beurre de cacao ou des glycérides semi-synthétiques.

Les topiques se présentent notamment sous forme de pommades.

Les médicaments selon l'invention sont particulièrement utiles en thérapeutique humaine pour leur action analgésique, et éventuellement antiinflammatoire et anti-pyrétique. Ils sont notamment indiqués pour le traitement des douleurs aiguës et chroniques, des algies rhumatismales et traumatiques, des douleurs dentaires, neurologiques et viscérales, des algies diverses (douleurs des cancéreux), des maladies inflammatoires (spondylarthrite ankylosante, rhumatisme articulaire aigu, arthrose) et éventuellement des états fébriles.

En thérapeutique humaine, les doses dépendent de l'effet recherché et de la durée du traitement; elles sont généralement comprises entre 100 et 2000 mg par jour pour un adulte.

D'une façon générale, le médecin déterminera la posologie qu'il estime la plus appropriée en fonction de l'âge, du poids et de tous autres facteurs propres au sujet à traiter.

Les exemples suivants, donnés à titre non limitatif, illustrent une composition selon l'invention.

Exemple A

On prépare selon la technique habituelle des comprimés dosés à 100 mgg de produit actif ayant la composition suivante:

| | |
|---|---|
| thioxo-5 tétrahydro-2,3,5,10b thiazolo [3,2-c] benzoxazine-1,3 | 0,100g |
| amidon | 0,110 g |
| silice précipitée | 0,035 g |
| stéarate de magnésium | 0,005 g |

Exemple B

On prépare une solution contenant 110 mg/cm3 de sel de sodium de carboxy-3 thioxo-5 tétrahydro-2,3,5,10b thiazolo [3,2-c] benzoxazine-1,3 dans de l'eau pour préparations injectables. La solution obtenue est répartie aseptiquement en ampoules à raison de 5 cm3 par ampoule. Les ampoules sont scellées et contiennent chacune 0,5 g de carboxy-3 thioxo-5 tétrahydro-2,3,5-10b thiazolo [3,2-c] benzoxazine-1,3.

**Revendications pour les Etats contractants: BE CH DE FR GB IT LI LU NL SE**

1. Un dérivé de la thiazolo [3,2-c] benzoxazine-1,3 caractérisé en ce qu'il répond à la formule générale:

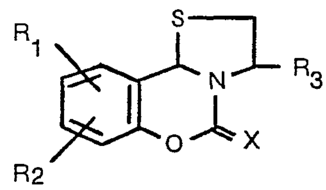

(I)

dans laquelle:

X représente un atome d'oxygène ou de soufre,

les symboles $R_1$ et $R_2$, qui sont identiques ou différents, représentent chacun un atome d'hydrogène ou d'halogène ou un radical alcoyle, alcoyloxy, alcoylthio ou trifluorométhyle en position -7, -8, -9 ou -10 et $R_3$ représente un atome d'hydrogène ou un radical carboxy,

les radicaux et portions alcoyle étant droits ou ramifiés et contenant 1 à 4 atomes de carbone ainsi que ses sels métalliques et ses sels d'addition avec les bases organiques azotées lorsque $R_3$ représente un radical carboxy, sous ses formes diastéréoisomères et leurs mélanges.

2. Un procédé de préparation d'un produit selon la revendication 1, caractérisé en ce que l'on fait agir un produit de formule générale:

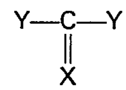

(dans laquelle X est défini selon la revendication 1 et les symboles Y, qui sont identiques, représentent des atomes de chlore ou des radicaux imidazolyle, benzimidazolyle, triazolyle, benzotriazolyle ou pyrazolyle) sur un dérivé de formule générale:

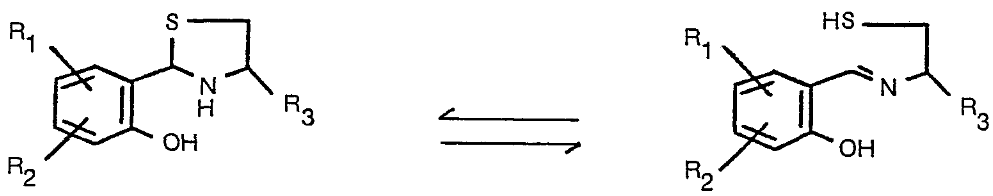

(III)

dans laquelle $R_1$, $R_2$ et $R_3$ sont définis selon la revendication 1, puis transforme éventuellement le produit obtenu en un sel métallique ou en un sel d'addition avec une base azotée lorsque $R_3$ représente un radical carboxy.

3. Médicament caractérisé en ce qu'il comprend au moins un produit selon la revendication 1 à l'état pur ou sous forme de compositions pharmaceutiques en association avec un ou plusieurs diluants ou adjuvants compatibles et pharmaceutiquement acceptables.

**Revendication pour l'Etat contractant: AT**

Procédé de préparation d'un dérivé de la thiazolo[3,2-c] benzoxazine-1,3 répondant à la formule générale:

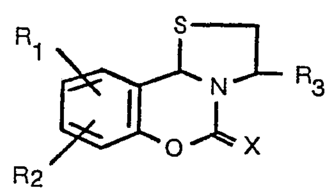

(I)

dans laquelle:

X représente un atome d'oxygène ou de soufre,

les symboles $R_1$ et $R_2$, qui sont identiques ou différents, représentent chacun un atome d'hydrogène ou d'halogène ou un radical alcoyle, alcoyloxy, alcoylthio ou trifluorométhyle en position -7, -8, -9 ou -10 et

$R_3$ représente un atome d'hydrogène ou un radical carboxy,

les radicaux et portions alcoyle étant droits ou ramifiés et contenant 1 à 4 atomes de carbone

ainsi que ses sels métalliques et ses sels d'addition avec les bases organiques azotées lorsque $R_3$ représente un radical carboxy, sous ses formes diastéréoisomères et leurs mélanges, caractérisé en ce que l'on fait agir un produit de formule générale:

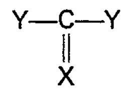

(dans laquelle X est défini selon la revendication 1 et les symboles Y, qui sont identiques, représentent des atomes de chlore ou des radicaux imidazolyle, benzimidazolyle, triazolyle, benzotriazolyle ou pyrazolyle) sur un dérivé de formule générale:

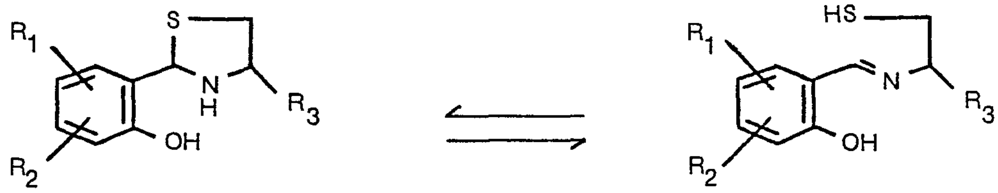

(III)

dans laquelle $R_1$, $R_2$ et $R_3$ sont définis selon la revendication 1, puis transforme éventuellement le produit obtenu en un sel métallique ou en un sel d'addition avec une base azotée lorsque $R_3$ représente un radical carboxy.

**Patentansprüche für die Vertragsstaaten: BE CH DE FR GB IT LI LU NL SE**

1. Ein Derivat des Thiazolo-[3,2-c]-benzoxazins-1,3, dadurch gekennzeichnet, daß es der allgemeinen Formel

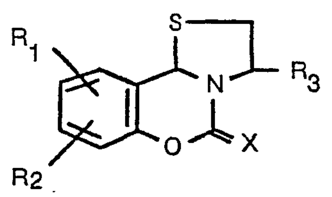

(I)

entspricht, worin:

X ein Sauerstoff- oder Schwefelatom bedeutet,

die Symbole $R_1$ und $R_2$, welche identisch oder verschieden sind, jeweils ein Wasserstoff- oder Halogenatom bedeuten oder einen Alkyl-, Alkoxy-, Alkylthio- oder Trifluormethylrest in 7-, 8-, 9- oder 10-Stellung und

$R_3$ ein Wasserstoffatom oder einen Carboxyrest bedeutet, wobei die Alkylreste und Alkylteile gerade oder verzweigt sind und 1 bis 4 Kohlenstoffatome enthalten, sowie seine Metallsalze und seine Additionssalze mit organischen Stickstoffbasen, wenn $R_3$ einen Carboxyrest bedeutet, in seinen diastereoisomeren Formen und deren Gemische.

2. Verfahren zur Herstellung eines Produkts gemäß Anspruch 1, dadurch gekennzeichnet, daß man ein Produkt der allgemeinen Formel

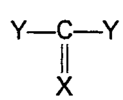

(worin X gemäß Anspruch 1 definiert ist und die Symbole Y, welche identisch sind, Chloratome oder Imidazolyl-, Benzimidazolyl-, Triazolyl-, Benzotriazolyl- oder Pyrazolylreste bedeuten) auf ein Derivat der allgemeinen Formel

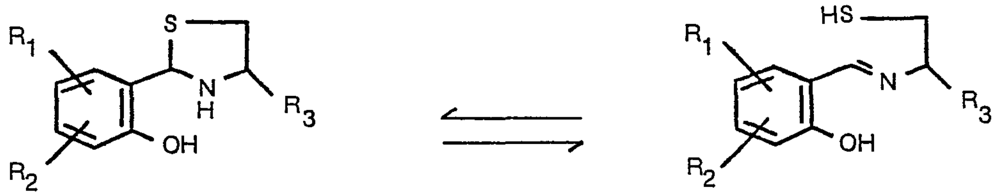

(III)

worin $R_1$, $R_2$ und $R_3$ wie in Anspruch 1 definiert sind, einwirken läßt und daß man dann gegebenenfalls

das erhaltene Produkt in ein Metallsalz oder ein Additionssalz mit einer Stickstoffbase überführt, wenn $R_3$ einen Carboxyrest bedeutet.

3. Arzneimittel, dadurch gekennzeichnet, daß es wenigstens ein Produkt gemäß Anspruch 1 in reinem Zustand oder in Form pharmazeutischer Zusammensetzungen zusammen mit einem oder mehreren verträglichen und pharmazeutisch annehmbaren Verdünnungs- oder Hilfsmitteln umfaßt.

**Patentanspruch für den Vertragsstaat: AT**

Verfahren zur Herstellung eines Derivats von 1,3-Thiazolo[3,2-c]benzoxazin der allgemeinen Formel

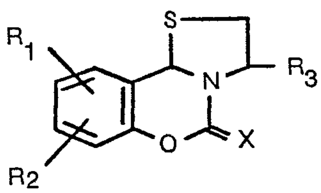

(I)

worin X ein Sauerstoffatom oder Schwefelatom darstellt, die Symbole $R_1$ und $R_2$, die gleich oder verschieden sind, jeweils ein Wasserstoffatom oder halogenatom oder ein Alkyl-, Alkyloxy-, Alkylthio- oder Trifluoromethylrest in 7-, 8-, 9- oder 10-Stellung sind und $R_3$ ein Wasserstoffatom oder ein Carboxyrest ist, wobei die Alkylreste und -teile gerad- oder verzweigtkettig sind und 1—4 Kohlenstoffatome enthalten, sowie seiner Metallsalze und Additionssalze mit stickstoffhaltigen organischen Basen, wenn $R_3$ ein Carboxyrest ist, in seinen diasterioisomeren Formen und ihren Mischungen, dadurch gekennzeichnet, daß man eine Verbindung der allgemeinen Formel

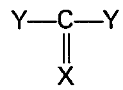

(worin X wie oben definiert ist und die Symbole Y, die gleich sind, Chloratome oder Imidazolyl-, Benzimidazolyl-, Triazolyl-, Benzotriazolyl- oder Pyrazolylreste sind) mit einem Derivat der allgemeinen Formel

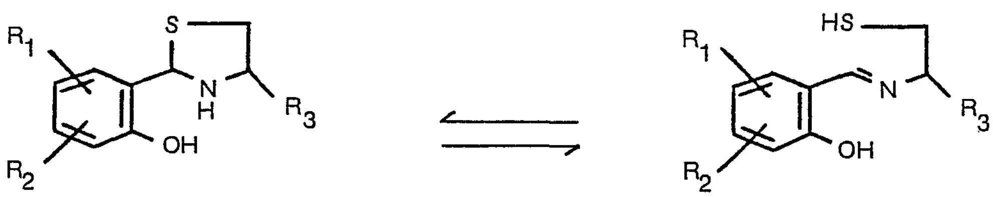

(III)

worin $R_1$, $R_2$ und $R_3$ wie oben definiert sind, umsetzt, worauf man gegebenenfalls die erhaltene Verbindung in ein Metallsalz oder ein Additionssalz mit einer stickstoffhaltigen Base überführt, wenn $R_3$ ein Carboxyrest ist.

**Claims for the Contracting States: BE CH DE FR GB IT LI LU NL SE**

1. A thiazolo[3,2-c]-1,3-benzoxazine derivative, characterised in that it corresponds to the general formula:

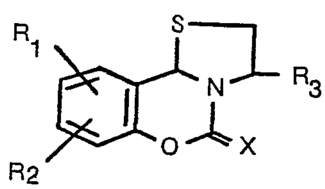

(I)

in which:

X represents an oxygen or sulphur atom, the symbols $R_1$ and $R_2$, which are identical or different, each represent a hydrogen or halogen atom or an alkyl, alkoxy, alkylthio or trifluoromethyl radical in the 7-, 8-, 9- or 10-position, and

$R_3$ represents a hydrogen atom or a carboxyl radical,

the alkyl radicals and portions being linear or branched and containing 1 to 4 carbon atoms, and also its metal salts and its addition salts with organic nitrogen bases if $R_3$ represents a carboxyl radical, in its diastereoisomeric forms and mixtures thereof.

2. A process for the preparation of a product according to Claim 1, characterised in that a product of the general formula:

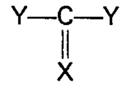

(in which X is defined according to Claim 1 and the symbols Y, which are identical, represent chlorine atoms or imidazolyl, benzimidazolyl, triazolyl, benzotriazolyl or pyrazolyl radicals) is reacted with a derivative of the general formula:

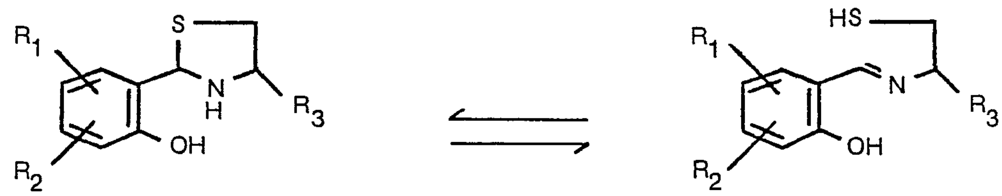

(III)

in which $R_1$, $R_2$ and $R_3$ are defined according to Claim 1, and the product obtained is then converted, if appropriate, to a metal salt or an addition salt with a nitrogen base if $R_3$ represents a carboxyl radical.

3. Medicament, characterised in that it comprises at least one product according to Claim 1, in the pure state or in the form of pharmaceutical compositions in association with one or more compatible and pharmaceutically acceptable diluents or adjuvants.

**Claim for the Contracting State: AT**

Process for the preparation of a thiazolo[3,2-c]-1,3-benzoxazine derivative corresponding to the general formula:

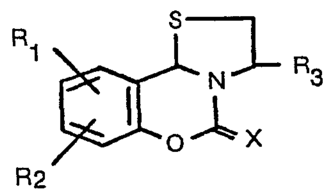

(I)

in which:

X represents an oxygen or sulphur atom,

the symbols $R_1$ and $R_2$, which are identical or different, each represent a hydrogen or halogen atom or an alkyl, alkoxy, alkylthio or trifluoromethyl radical in the 7-, 8-, 9- or 10-position, and

$R_3$ represents a hydrogen atom or a carboxyl radical, the alkyl radicals and portions being linear or branched and containing 1 to 4 carbon atoms,

and also its metal salts and its addition salts with organic nitrogen bases if $R_3$ represents a carboxyl radical, in its diastereoisomeric forms and mixtures thereof, characterised in that a product of the general formula:

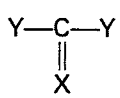

(in which X is defined as above and the symbols Y, which are identical, represent chlorine atoms or imidazolyl, benzimidazolyl, triazolyl, benzotriazolyl or pyrazolyl radicals) is reacted with a derivative of the general formula:

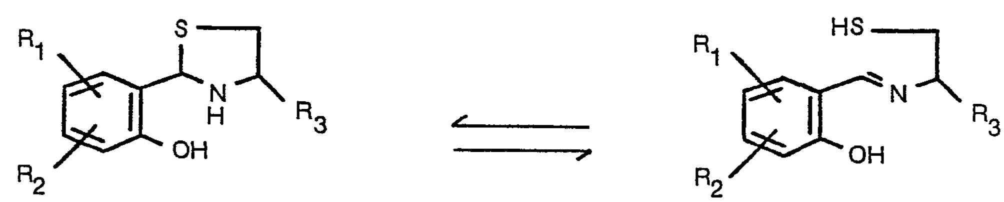

(III)

in which $R_1$, $R_2$ and $R_3$ are defined as above, and the product obtained is then converted, if appropriate, to a metal salt or an addition salt with a nitrogen base if $R_3$ represents a carboxyl radical.